# EUROPEAN PATENT SPECIFICATION

(11) **EP 0 327 522 B1**
(45) Date of publication and mention of the grant of the patent: **13.12.1995**
(21) Application number: 89850031.9
(22) Date of filing: 02.02.1989
(51) Int. Cl.: C07K 14/315, C07K 14/765, C12P 21/02, C12N 1/20, C12N 11/02

(54) **Production of fused proteins or polypeptides**
Herstellung von Fusionsproteinen oder -polypeptiden
Production de protéines ou polypeptides de fusion

(30) Priority: 05.02.1988 SE 8800378
(43) Date of publication of application: 09.08.1989
(73) Proprietor: Nygren, Per-Ake, S-122 51 Enskede (SE); Abrahmsen, Lars, 111 31 Stockholm (SE); Uhlen, Mathias, S-752 39 Uppsala (SE)
(72) Inventor: Nygren, Per-Ake, S-122 51 Enskede (SE); Abrahmsen, Lars, 111 31 Stockholm (SE); Uhlen, Mathias, S-752 39 Uppsala (SE)
(74) Representative: Henningsson, Gunnar

(56) References cited:
- WO-A-87/05631
- MOLECULAR IMMUNOLOGY, vol. 24, no. 10, 1986, pages 1113-1122; L. BJÖRK et al.
- THE EMBO JOURNAL, vol. 5, no. 7, 1986, pages 1567-1575; B. GUSS et al.
- JOURNAL OF MOLECULAR RECOGNITION, vol. 1, no. 2, September 1988, pages 69-74; P.-A. NYGREN et al.

## Description

The present invention relates to a method of preparing protein and polypeptide products with high purity through recombinant DNA technology, and more particularly to the utilization of such technology to prepare novel gene products comprising desired proteins or polypeptides, which novel gene products are easily refinable, and optionally converting such gene products into the desired proteins or polypeptides. More specifically, the invention covers a method of producing a fusion protein or polypeptide capable of selective binding to serum albumin.

Gene fusion is a procedure wherein the coding sequence of two or more genes are spliced together to form a combined gene which on expression in a suitable host organism will produce a fusion product wherein the separate proteins or polypeptides coded for by the respective genes are fused together into a single molecule.

The present invention provides means to facilitate immobilization and purification of proteins through a method based upon recombinant DNA technology which permits desired proteins and polypeptides to be produced with extreme purity. According to the invention this is achieved by utilizing the specific albumin-binding properties of protein G from Streptococcus strain G148 in combination with gene fusion technology as will be explained below.

In accordance with the present invention gene fusion is used to combine a first DNA-sequence coding for albumin-binding part with a second DNA-sequence coding for a desired protein or polypeptide into a functional gene capable of expressing the fusion product of said desired albuminbinding part. Due to the albumin-binding ability the produced protein or polypeptide can easily be isolated with high efficiency by conventional affinity chromatography utilizing serum albumin of the type human serum albumin (HSA) immobilized to a suitable carrier. The carrier-bound fusion product may be used as such, e.g. if the desired protein is an enzyme, or it may be released from the carrier, either as a whole including the albumin-binding part, or only the desired protein or polypeptide part thereof through cleavage with a suitable agent.

A basic aspect of the present invention is thus the provision of a recombinant DNA cloning vehicle or vector comprising a DNA sequence coding for a desired protein or polypeptide operatively linked to a DNA sequence coding for albumin-binding part, such that said DNA sequences together code for an albumin-binding fusion product of said desired protein or polypeptide and said albumin-binding part. In order to be capable of transforming (which is also meant to include the case that the vector is a bacteriophage) a host organism to produce said fusion product, the vector in conventional manner further comprises a replicon and a promotor for the combined fusion product coding DNA sequence. For purposes which will be further elucidated below said combined DNA sequence may comprise a sequence coding for an appropriate cleavage site between the DNA sequences coding for the desired protein and albumin-binding part, respectively, such that the albumin-binding part of the fusion molecule may be cleaved off as mentioned above.

By transforming a compatible host organism with said vector to permit expression of the above combined DNA sequence and culturing the host in a nutrient medium the corresponding albumin-binding fused protein or polypeptide will be produced. Although bacterial hosts, such as strains of, for example, Escherichia, Bacillus and Staphylococcus, are preferred for the purposes of the invention, it is, of course, also within the scope thereof to use other hosts, such as yeasts and other fungi, plant cells in culture, etc. The transformation of the hosts may be effected with well-known methods.

Due to albumin-binding ability of the protein G moiety of the fusion molecule produced by the cultured host-organism the fusion molecule can be very efficiently isolated from the cell culture by means of HSA immobilized to a suitable carrier. If the fusion product is secreted into the surrounding medium the binding to the carrier may be performed directly from the medium. If, on the other hand, the fusion product remains within the cells the latter have to be ruptured before such binding can be effected. Rupture of the cell walls may be effected in conventional manner by, e.g., high pressure, ultrasonication, homogenization, shaking with glass-beads etc. In cases where the product is trapped within the periplasmic space between two cell membranes, as in gram-negative bacteria, an osmotic shock procedure may be used to release the product into the suspension medium. Any other treatment of the cultured cells or the growth medium prior to the HSA-aided isolation of the fusion product is, of course, also within the scope of the invention.

In conventional manner the immobilization process may be performed batch-wise with the HSA-coupled carrier slurried in a suitable medium or on a column of the activated carrier. Any conventional carrier material to which HSA can be sufficiently coupled for the present purposes may be used. The methods for coupling or immobilizing HSA to such carrier materials is well-known and need not be described in any detail herein. It is possible to use adsorption of HSA to surfaces of microtiter wells as a means for coating.

Release or desorption of the fused protein or polypeptide which is bound to the HSA-carrier may be effected by conventional methods, such as lowering the pH, e.g. by means of acetic acid buffer (pH 2.7), treatment with high salt concentrations or chaotrophic ions, or by competitive elution using excess soluble HSA to displace the fusion protein or polypeptide from the HSA-carrier adsorbent. The choice of desorption method should, of course, be made with regard to the particular desired protein or polypeptide, such that a desired activity thereof is not lost or considerably reduced thereby. From the resulting eluate the fusion protein or polypeptide may readily be isolated and, if desired, subjected to further purification steps, such as gel filtration, ion exchange etc.

The purified protein or polypeptide obtained may in itself be a valuable product as will be described below, and another aspect of the present invention is therefore the provision of a method of producing a highly purified fused protein or polypeptide product comprising the steps of transforming a compatible host with the above vector, culturing said host in a nutrient medium, isolating said fused protein or polypeptide from said host culture by selective binding thereof to an HSA-supporting carrier, and optionally releasing the fused protein or polypeptide from the carrier, as well as such an isolated fused product obtained thereby.

One valuable use of such a fusion product is when the protein fused to the albumin-binding part is an enzyme. In such cases the HSA-binding activity of the fusion product is utilized for immobilizing the enzyme to a carrier material having HSA coupled thereto. Such an enzyme reactor system offers several advantages. Since the enzyme is bound to the carrier via the HSA coupling, all the enzyme molecules will be bound to the carrier in exactly the same way and maximum activity thereof will thus be obtained. When the enzyme activity has decreased to an unacceptably low level, such a system can easily be regenerated by conventionally desorbing the enzyme from the carrier through a pH change, e.g. lowering the pH and then binding fusion product containing active enzyme thereto. The binding or adsorption of the fused enzyme in question to the HSA-coupled carrier may be effected either directly from the appropriately pre-treated cells or cell medium, or in purified state after adsorption and desorption from another HSA-coupled adsorbent.

Another valuable use of such a fusion protein is for the directed immobilization of an antigen used in diagnostic tests, such as ELISA, in particular for assays to determine the presence and/or quantity of a specific antibody towards the said antigen. Using the present invention, the antigen comprising a peptide or a protein fused to the albumin-binding part being immobilized to a surface coated with HSA. Analysis of the amount of specific antibodies towards the antigen moiety can then be performed by known methods involving labelled protein A or anti-antibodies. Such a fusion protein directed immobilization of the peptide or protein antigen offers several advantages. All molecules will be bound in the same way and no purification of the fusion protein prior to the immobilization operation is needed, since proteins lacking the albumin-binding part may be washed away. In addition, the immobilization does not need any chemical coupling and the antigen-solid support system can be regenerated simply by lowering the pH to desorb bound fusion protein from the HSA coated to the surface.

Yet another valuable use of such a fusion protein is to convert a HSA-matrix to a more specific affinity matrix using the present invention to immobilize a peptide or protein ligand fused to the albumin-binding part. In such a way, the HSA-column can be used to purify specific antibodies towards an antigen from human or animal serum. This offers advantages when monoclonal or polyclonal antibodies directed to a specific antigen need to be concentrated or eliminated. The latter can be important in extracorporal therapy for a patient having autoantibodies towards biologically important proteins.

The albumin-binding protein part of the fused protein or polypeptide may under certain conditions be cleaved off, the pure desired protein or polypeptide thereby being obtained. In another aspect the present invention therefore provides a method of producing a desired protein or polypeptide of high purity comprising the steps of transforming a compatible host with the above mentioned vector, culturing said host in a nutrient medium, isolating said fused protein or polypeptide from the cell culture by selective binding to a HSA-supporting carrier, and cleaving off the desired protein or polypeptide from the albumin-binding part of said fused protein or polypeptide, either directly from the carrier bound fusion product or after desorption thereof from the carrier.

A necessary condition to permit such cleavage of the fused protein or polypeptide is, of course, that it contains a unique cleavage site which may be recognized and cleaved by suitable means. Such a cleavage site may be a unique amino acid sequence recognizable by chemical or enzymatic means and located between the desired protein or polypeptide and albumin-binding part, respectively, of the fused product to be produced. Such a specific amino acid sequence must not occur within the desired protein or polypeptide and preferably not in the binding part of the fusion product. Examples of enzymatic agents include proteases, such as factor Xa, which in some cases recognizes the amino acid sequence NH₂-Ile-Glu-Gly-Arg-COOH ; chymosin (rennin), which cleaves the Met-Phe bond; kallikrein B, which cleaves on the carboxyl side of Arg in X-Phe-Arg-Y; enterokinase, which recongizes the sequence X-(Asp)ₙ-Lys-Y, wherein n=2-4, and cleaves it on the carboxyl side of Lys; thrombin which cleaves at specific arginyl bonds. Examples of chemical agents include cyanogen bromide (CNBr), which cleaves after Met; hydroxylamine, which cleaves the Asn-Gly bond, formic acid, which in high concentration (∼70%) specifically cleaves Asp-Pro.

As appears from the above a crucial part of the present invention is the provision of the recombinant DNA structure or vector comprising the combined gene coding for the present fusion protein or polypeptide and capable of transforming a host cell to permit expression thereof and production of the fusion product. The present invention is meant to encompass any such vector irrespective of how it has been obtained using, for example, various restriction enzyme cutting, ligating, transforming and screening techniques well-known in the art as well as any appropriate vector materials and host-organisms. Thus, the DNA sequence coding for the desired protein or polypeptide may be inserted into a suitable vector and the albumin-binding coding DNA sequence inserted subsequently, or vice versa: or the two DNA sequences may be introduced simultaneously into the vector. It is also possible to insert the respective DNA sequences in parts thereof into the vector. Further the two DNA sequences may be arranged with either the albumin-binding coding sequence or the sequence coding for the desired protein or polypeptide at the 5'-end or start of the combined gene. The special techniques for accomplishing such insertions and combinations with maintained correct reading frames, including the provision of suitable restriction sites therefore, are well-known per se in the art.

The invention also involves a recombinant DNA molecule comprising the recombinant DNA sequence as described above and fused 3' thereof at DNA level a production gene. By this arrangement such molecule obtains the ability to express a fused protein in a suitable host. Such production gene may be that of a somatomedin, examples of which are: growth hormones or factors, such as hGH (Human Growth Hormone), IGF-I, IGF--II, NGF (Nerve Growth Factor), EGF (Epithermal Growth Factor) and PDGF (Platelet Derived Growth Factor). The production gene may also be one coding for an interferon, interleu Kin-2, insulin, neuropeptide, gastrointestinal peptides, tissue plasminogen activator (tPA) and active derivatives thereof, etc. The production gene may also code for a structural gene for an enzyme or parts thereof.

According to still another aspect of the invention there is provided a process for cleaving a fused protein expressed in a biological system by the recombinant DNA molecule as defined above.

Finally, the invention involves a plasmid vector comprising the recombinant DNA molecule as described above. The invention also involves bacterial cells harbouring the recombinant DNA molecule defined above. The molecule can be inserted in the chromosome of the bacterial cell but may also be contained in a plasmid vector.

The host cell is for example Gram negative and is particularly constituted by an E.coli.

The invention will in the following be further illustrated by non-limiting examples with reference to the appended drawings wherein:
Fig. 1 is a schematic drawing of the streptococcal protein G gene (G) and the constructs containing fragments of this gene;
Fig. 2 shows an analysis by gel electrophoresis of the growth medium of E.coli cells containing plasmid pEG (Fig 1B). Lane 1, total proteins from clarified growth medium; lane 2, IgG-affinity purified proteins; lane 3, HSA-affinity purified proteins;
Fig. 3 shows an analysis by gel electrophoresis of IgG-and HSA-binding of fragments expressed from two subcloned fragments.
   Growth media from E.coli cells, containing the different constructs, were purified on HSA- or IgG-Sepharose. Eluted proteins were lyophilized and analyzed on SDS-PAGE.
   A; cells containing pB1B2 (Fig. 1D), lane 1, IgG-affinity purified proteins (note, the light and heavy chains of IgG); lane 2, HSA-affinity purified proteins.
   B; cells containing pC2C3 (Fig. 1A), lane 1, IgG-affinity purified proteins; lane 2, HSA-affinity purified proteins.

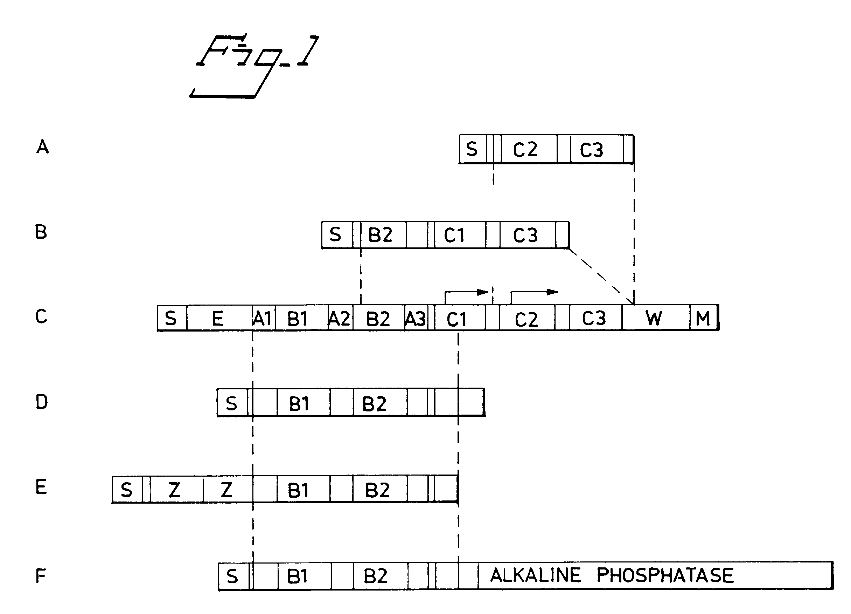

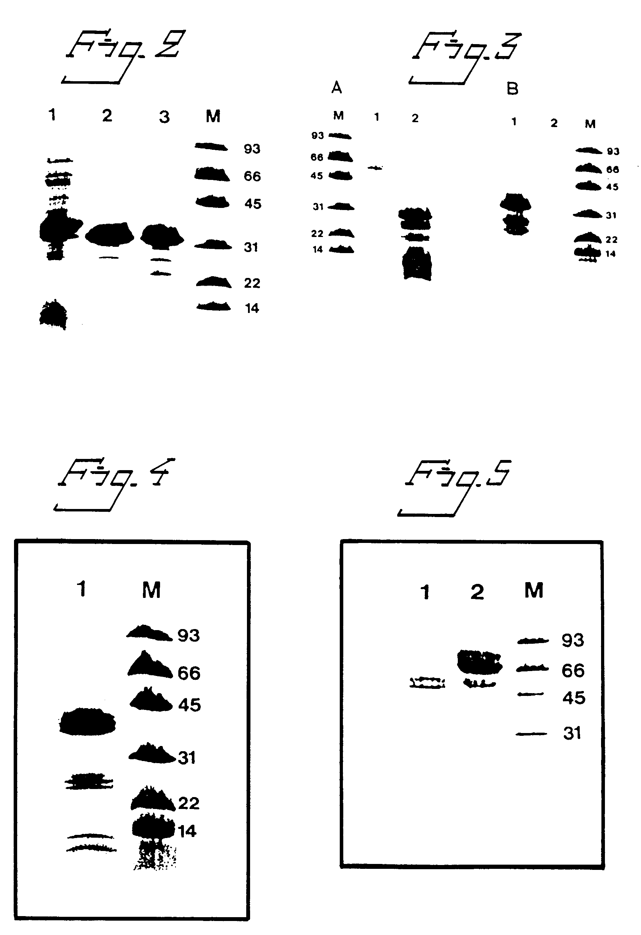

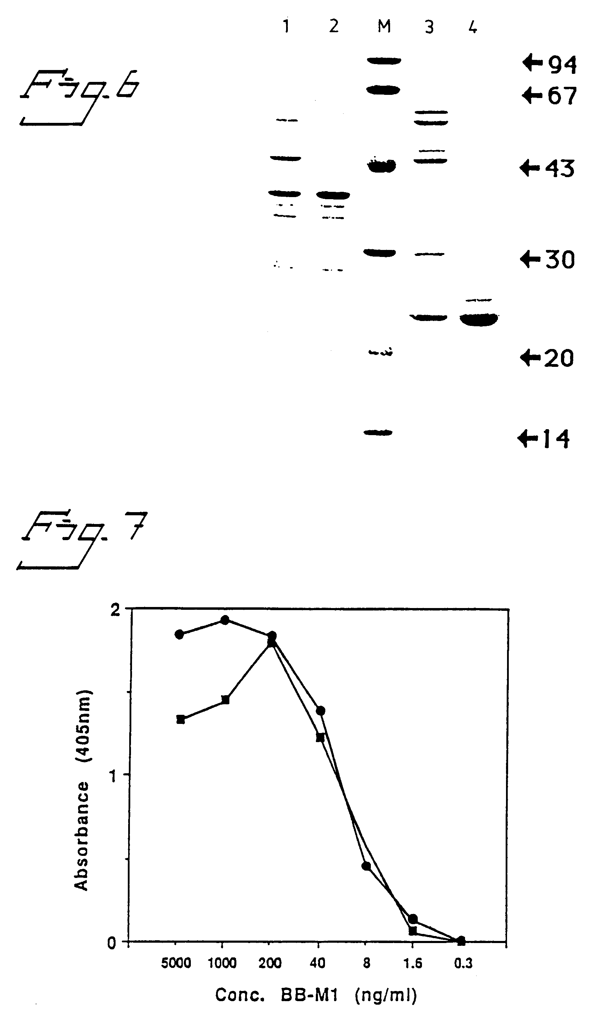

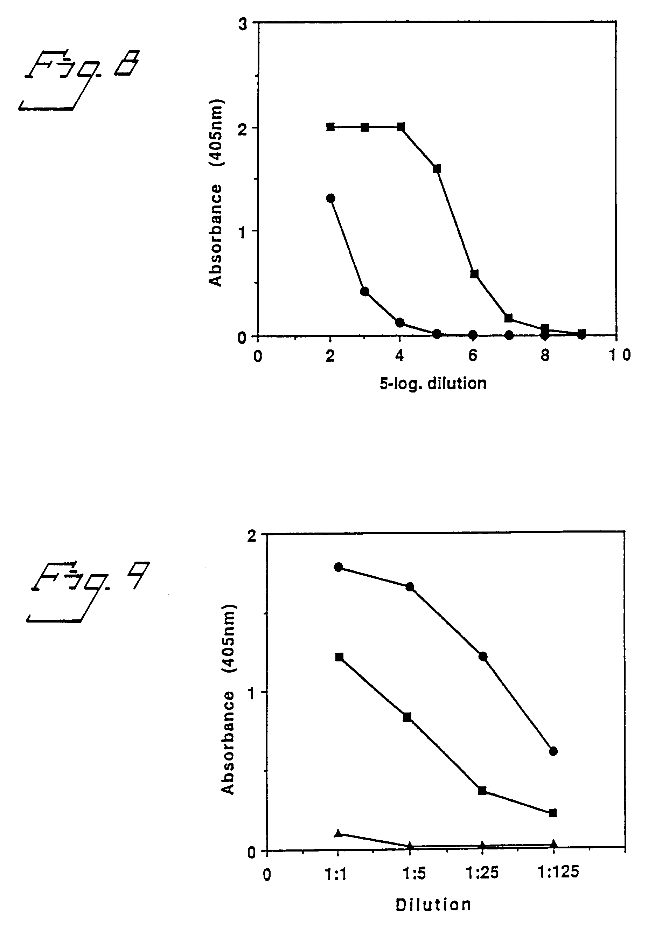

Fig. 4 shows an analysis by gel electrophoresis of HSA--affinity purified material from the growth medium of cells containing plasmid pZZB1B2 (Fig. 1E).

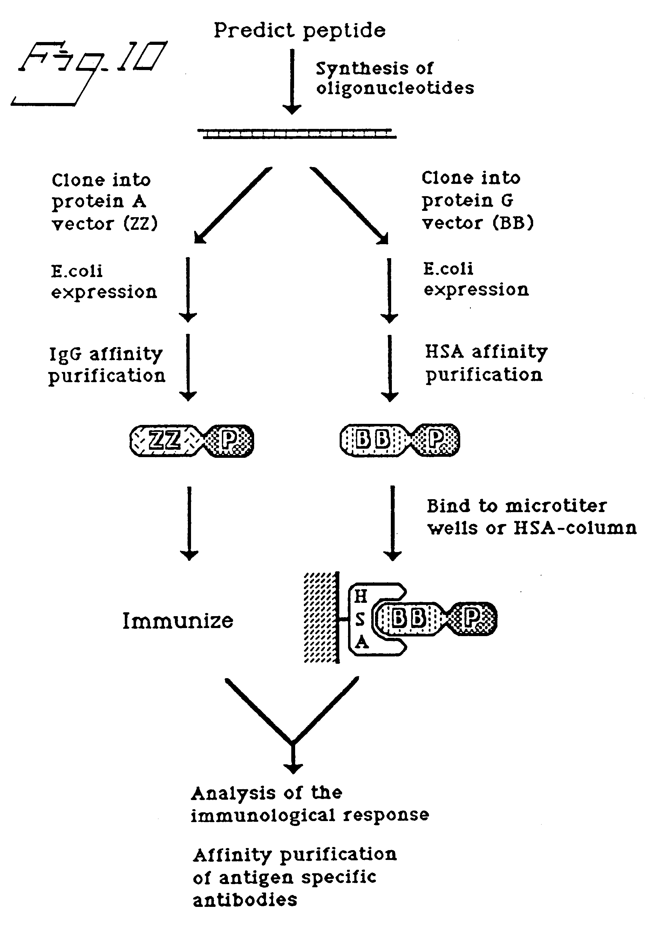

Fig. 5 shows an analysis by gel electrophoresis of purified material obtained by osmotic shock of E.coli strains pASP (lane 1) and pNP-1 (lane 2) purified with ion exchange and HSA-affinity chromatography, respectively.
Fig. 6 shows an SDS/PAGE analysis of the fusion proteins on a 13% polyacrylamide gel under reducing conditions. Lane 1: total periplasmic content of E.coli cells harboring pB1B2M1; lane 2: BB-M1 protein affinity purified on HSA-Sepharose; lane 3: total periplasmic content of E.coli cells harboring pEZZM1; lane 4: ZZ-M1 protein affinity purified on IgG-Sepharose. Numbers indicate approximate molecular weights in kDa.
Fig. 7 shows a titration in ELISA of the fusion protein BB-M1 bound to HSA-coated microtiter wells (□). The microtiter plates were incubated with antiserum diluted 1:2000 from a rabbit immunized with the fusion protein ZZ-M1 in CFA. Values were reduced by subtracting the reactivity of the serum with the protein G-derived part of BB-M1 (OD, 0.022-0.088).
Fig. 8 shows the reactivity in ELISA with the fusion protein BB-M1 of antisera from a rabbit immunized with the fusion protein ZZ-M1 in CFA (■) or without adjuvant (●). Values were reduced by subtracting the reactivity of the sera with protein G-derived part of BB-M1 (OD, 0.011-0.158). Absorbance>0.1=positive.
Fig. 9 is the result of affinity purification on BB-M1 of antiserum from a rabbit immunized with ZZ-M1 in CFA. Reactivity in ELISA with BB-M1 of the applied IgG fraction (●), the flow-through fraction (▲) and the fraction eluted with 0.2 M glycine buffer (■). Fractions were adjusted to comparative volumes with regard to the dilution caused by the purification process.
Fig. 10 is a schematic diagram showing the basic concept of the dual expression system. ZZ: IgG-binding region derived from SpA; BB: serum albumin binding domain from streptococcal protein G; P: immunogenic peptide; HSA: human serum albumin. See text for details.

Specific embodiments of the invention will now be described in detail.

### Starting materials

E.coli RRI del M15 [Langey et al, Proc.Natl.Acad.Sci., USA, 72, 1254-1257 (1975)] were used in the Examples. The cloning vehicles used were pUC8 [Viera and Messig, Gene, 19, 259-268 (1982)], pEZZ8 [Löwenadler et al, Gene 58, 87-97 (1987)], pEMBL8- [Dente et al, Nucl.Acids Res. 11, 1645-53, (1983)], pASP [Abrahmsén et al, EMBO J. 4, 3901-3906 (1985)], pCH39 [Hoffman and Wright, Proc.Natl.Acad.Sci., USA, 82, 5107-5111 (1985)] and M13mp18 (commercially available from Pharmacia, Uppsala, Sweden). All the strains and vectors are available at the Department of Biochemistry, Royal Institute of Technology, Stockholm, Sweden. The plasmid vector pNP-1 has been deposited with the Deutsche Sammlung von Mikroorganismen (DSM) Braunschweig, Federal Republic of Germany, under No. DSM-4386 on February 3, 1988.

### Buffers and Media

PBST: 8.0 g NaCl, 0.2 g KH₂PO₄, 2.9 g Na₂HPO₄ X 12H₂O, 0.2 g KCL, 0.2 ml Tween® 20 and 0.2 g NaN₃ made up to 1 litre with distilled H₂O (pH 7.4).
TSB: 30 g Tryptic Soy Broth, made up to 1 litre and autoclaved.
TST: TRIS (25mM) and HCl to pH 7.4, 200 mM NaCl, 0.05 % Tween® 20.

### Routine Methods

Methods used routinely in molecular biology are not described (like the use of commercial restriction enzymes, DNA-ligations, Bal 31 exonuclease, S1 nuclease and Klenow polymerase, transformation of E.coli and isolation of plasmid DNA).

The osmotic shock procedure described by Nossal and Heppel, J. of Biol. Chem., vol. 244, No.13, pp 3055-3062 (1966) involves exposal of the cells to 20% sucrose with 0.1 mM EDTA followed by dispersal in cold 5·10⁻⁴ M MgCl₂ which causes the periplasmic content to be liberated.

In order to analyze protein fractions by SDS-PAGE using the PHAST-system (Pharmacia, Uppsala, Sweden), the samples were dissolved in loading buffer [2.5% SDS, 5% Dithiothreitol (DTT) and 0.01% Bromphenol blue]. Gradient (8-25%) polyacrylamide gels with 5% SDS were run at 10 mA for 30 min. and subsequently stained with Coomassie-blue.

### EXAMPLE I

### Cloning and expression of the albumin-binding fragment of streptococcal protein G

A schematic drawing of the gene encoding protein G from Streptococcus strain G148, as described by Olsson et al (Eur.J. of Biochem. 168: 319-324 (1987)), is shown in Fig. 1C. Different gene fragments encoding parts of the gene were used to assemble the three constructs schematically outlined in Fig. 1A, B and D. The construction of these constructs are described in the following.

The coding sequence of the three IgG binding regions from protein G is contained within the ThaI-BstNI fragment of the gene between nucleotides 1,021 and 1,852 (Olsson et al (1987) Eur.J.Biochem. 168: 319-324). The ThaI site was converted to a BamHI site by ligation of a synthetic oligonucleotide BamHI-linker (5′-CGGATCCG-3′) and the BstNI was made blunt ended using Klenow polymerase to fill in the sticky end. This fragment was then cloned into the multilinker of pTZ18R (Pharmacia, Sweden). This vector had first been cleaved with XbaI followed by Klenow polymerase treatment to fill in the sticky end followed by digestion with BamHI. The resulting plasmid was called pUG26. To prevent read through into the β-galactosidase gene a synthetic oligonucleotide giving a translational stop was cloned into the PstI site. Since the protein G gene contains two PstI sites, the following scheme was used. Plasmid pTZ18R was cleaved with PstI, treated with T4-polymerase to generate blunt ends and ligated to a universal translation linker, UTL (Pharmacia, Sweden). The construct was digested with EcoRI and SalI and ligated to the isolated 860 base pairs EcoRI-SalI fragment from pUG26, to yield plasmid pUG27. The BamHI-HindIII fragment from pUG27 was inserted into pEZZ8 giving plasmid pEZZG with an in-frame fusion to two synthetic IgG binding domains based on protein A. The two ZZ fragments were removed by cleavage with AccI and religation. Analysis of the obtained plasmid revealed that a homologous recombination had occurred between the C1 and C2 regions. This yielded the plasmid pEG with the protein A signal peptide fused in-frame to the IgG binding domains C1 and C3 of the protein G gene. This truncated receptor is schematically shown in Fig. 1B.

The plasmid pUG27 was digested with HindIII and partially with ClaI and a gene fragment encoding regions C2 and C3 was purified on an agarose gel. This fragment was subcloned between AccI and HindIII in the plasmid pUC8.

By the use of the EcoRI site close to the AccI site in the mp8 multilinker of pUC8, and the HindIII site, the same fragment was cleaved out. It was inserted into the purified vector fragment of pEG, previously digested with EcoRI and HindIII, to give the vector pC2C3 schematically shown in Fig. 1A.

In order to subclone the AB-region, the plasmid pSPG2 (Guss et al, 1986) was digested with EcoRI, followed by treatment with Klenow polymerase to fill in the sticky ends. A synthetic SalI-linker (GGTCGACC, Pharmacia, Sweden) was added by ligation. Digestion with SalI and PstI released a 640 bp fragment which was isolated from an agarose gel and inserted between the same sites in the positive selection vector pEMBL8-. The protein G gene fragment was cut out of this plasmid using the EcoRI site and the HindIII site, close to either insertion site, and inserted into the vector fragment of pEG, as above. The resulting vector, pB1B2 (Fig. 1D), encodes part of protein G from the last three residues of region E to the first 24 of region C1, preceded by the control sequences of SPA.

For affinity chromatography, IgG-Sepharose was obtained from Pharmacia (Uppsala, Sweden) and HSA-Sepharose was prepared by coupling purified HSA (Kabi-Vitrum, Stockholm, Sweden) to CNBr-activated sepharose (Pharmacia, Uppsala, Sweden) as described by Axén et al (1967) Nature 214, 1302-1304. The HSA- and IgG-Sepharose were pushed into columns with about 4 ml of gel. The columns were equilibrated with PBST-buffer.

The E.coli strains containing plasmids pEG, pC2C3 and pB1B2 were cultivated in duplicate at 37°C overnight in 200 ml of TSB plus ampicillin (70 mg/l). The cells were pelleted by centrifugation at 6000 g for 10 minutes and the supernatants of the duplicates were loaded directly to the IgG- and the HSA-columns, respectively. The columns were thereafter washed with 10 column volumes of PBST-buffer and 1 volume of 5 mM NH₄Ac, pH 5.5, the latter to lower the buffer capacity which enables efficient elution. Elution was made with 0.5 M HAc pH 2.8 and fractions of 1 ml were collected. The OD at 280 nm measured and the relevant fractions (3 and 4) were collected, pooled and lyophilized

After lyophilization, the various fractions were dissolved in loading buffer (2.5% SDS, 5% Dithiothreitol [DTT] and 0.01% Bromphenol blue) and analysed by Phast electrophoresis (Pharmacia, Uppsala, Sweden) on gradient (8-25%) polyacrylamide gel with 5% NaDodecyl sulphate (SDS-PAGE) as described by the supplier.

For the E.coli strain containing pEG the proteins, before and after purification, was analysed by SDS-PAGE. As can be seen in Fig. 2, the recombinant SPG constitute more than 50% of the extracellular proteins (lane 1). Affinity chromatography on either IgG-Sepharose (lane 2) or HSA-Sepharose (lane 3) resulted high yield of pure recombinant protein, with less than 10% degradation. Note, that the truncated SPG has an abnormal migration rate giving an apparent size of approximately 33 kDa, which is considerably larger than the 22 kDa predicted from the base sequence. This is in accordance with earlier observations (Guss et al., 1986).

For the E.coli strains containing pC2C3 and pB1B2, the results of the electrophoresis of the affinity purified material is shown in Fig. 3. This shows that the 30.5 kDa protein encoded by pB1B2 does not bind to IgG-Sepharose (lane 1), but is efficiently bound to HSA (lane 2). In contrast, the 17.2 kDA protein encoded by pC2C3 bind to IgG-Sepharose, but not to HSA-Sepharose (Fig. 4, lanes 1 and 2).

These results demonstrate that the two receptor activities of protein G are associated with different parts of the primary structure and are functionally independent. The part responsible for the albumin-binding activity is composed of the homologous region B repeated twice, with one region A on either side, i.e. A-B-A-B-A (Fig. 1C). The last 64 amino acid of this part are included in the albumin-binding protein encoded by one of the constructs, pEG, i.e. most of the last B region and all of the last A region. Therefore, a relatively small fragment of native protein G is sufficient for HSA-binding.

### EXAMPLE II

### Purification of a fusion protein using the albumin-binding activity.

The expression vector pEZZT308 was made by insertion of a synthetic translation and transcription stop linker, based on trpT
(5′-AAGCTTAAGTAAGTAAGCCGCCAGTTCCGCTGGCGGCATTTTTTTTGATATCATCGA T-3′, KabiGen AB, Stockholm, Sweden), between HindIII and ClaI in the vector pEZZ8. To obtain pZZB1B2, the EcoRI-HindIII fragment from pB1B2 was inserted into this vector, previously digested with the same enzymes. This plasmid encodes a fusion protein between ZZ derived from staphylococcal protein A (Nilsson et al., 1987 Protein Engineering, 107-113) and the AB-region of SPG, preceded by the SPA promoter and signal peptide as schematically shown in Fig. 1E.

The E.coli strain containing pZZB1B2 was cultured in 200 ml TSB plus ampicillin (70 mg/l) at 37°C and the overnight culture was harvested and the cells were clarified by centrifugation at 6000 g for 10 minutes. The clarified medium was affinity purified by HSA-Sepharose as described above and the eluted material was analyzed by PHAST electrophoresis. As shown in Fig. 4, the fusion protein of 38.7 kDa could be bound and eluted from either of the columns.

These results demonstrate that a heterologous protein (ZZ) binds to albumin and can be purified by HSA-affinity chromatography when fused to the albumin-binding B1B2 part of protein G.

### EXAMPLE III

### Immobilization and purification of a fusion protein using the albumin-binding activity.

Plasmid pB1B2 was digested with restriction enzymes EcoRI and SalI, treated with Klenow polymerase and religated to yield pB1B2 R/S. A synthetic oligonucleotide (TGCAAGATCTTTCAATTTCCCTATCCTCGAGAATTCTAAGCTT and its complementary sequence) was inserted in pB1B2 R/S previously cleaved with PstI and HindIII, giving rise to plasmid pB1B2HIV resistant to PstI. A multipurpose cloning linker derived from M13mp18 was cloned between the EcoRI and HindIII restriction sites, resulting in expression of the LacZ′ gene positioned immediately downstream. The resulting plasmid was designated pB1B2HIVmp18.

In order to fuse the gene encoding alkaline phosphatase to the sequence encoding the albumin-binding protein, plasmid pCH39 was used as a source of the gene. Cleavage with PstI releases a 3 kbp. fragment containing the gene encoding alkaline phosphatase adapted for inframe fusion to pB1B2HIVmp18 previously digested with PstI. During the ligation, also the rest of pCH39 was also incorporated in a triple-ligation to yield apart from the desired fusion, a plasmid encoding resistance not only to ampicillin but also to tetracycline. Samples of E.coli cells containing this construct designated pNP-1 have been deposited at DSM in Braunschweig and given the accession No. DSM-4386.

As a control, an E.coli strain containing the plasmid pASP encoding a secreted alkaine phosphatase was used. The E.coli strains containing pNP-1 and pASP were separately cultivated in 200 ml of TSB supplemented with ampicillin (70 mg/l) and 0.9% KH₂PO₄ to suppress the chromosomal alkaline phosphatase gene. Proteins localized in the periplasmic space were released using an osmotic shock procedure according to known techniques.

The alkaline phosphatase activity released by this procedure was determined for the two samples by incubating with p-nitrophenylphosphate (Sigma 104-0) as described by the supplier. Enzymatic activity yields hydrolysis of the substrate releasing p-nitrophenol, which can be measured as the change of absorbance at 410 nm. A unit of activity was defined as the change of absorbance (410 nm) per minute for a sample volume corresponding to 1 ml of overnight culture.

To determine whether the fusion protein can be selectively immobilized to a HSA-containing matrix, the following test was performed. Three »l of the materials obtained from the osmotic shock procedures of the two strains were separately mixed with 0.5 ml TST and 50 »l of HSA-Sepharose for 30 minutes at room temperature. After centrifugation at 10.000 g for 1 minute, the phosphate activity in the supernatant was determined. The gel was washed twice with 1 ml of TST and the activity bound to the gel was measured as described above.

The results are shown in the follwing Table 1.

**TABLE 1**

| Plasmid | Total (U) | % activity not bound | % activity bound |
|---|---|---|---|
| pASP | 2.74 | 100 | < 0.1 |
| pNP-1 | 1.14 | 64 | 36 |

In the table "Total" gives the activity released by the osmotic shock procedure. The results show that the alkaline phosphatase from cells containing pASP (control) does not bind to HSA-Sepharose. In contrast, a large portion of the alkaline phosphatase fusion protein encoded by pNP-1 binds to the HSA-matrix. This demonstrates that the albumin-binding domain can be used to immobilize a heterologous protein with intact enzymatic activity.

The material obtained from the osmotic shock procedure of the two E.coli strains was separately affinity purified by HSA-Sepharose chromatography as described in Example I. No protein material was bound to the column using the periplasmic fraction above from the E.coli strain containing pASP (control) as determined by the absorbance at 280 nm of the eluted material. In contrast, the strain pNP-1 yielded bound protein that could be eluted with low pH. Analysis by PHAST electrophoresis of this material is shown in Fig. 5, lane 2. This reveals a major bond of the expected size (71 kDa) slightly larger than native alkaline phosphatase (lane 1) purified by ion-exchange and gel-filtration as described according to standard procedures.

These results demonstrate that a protein consisting of an active enzyme fused to the B1B2 albumin-binding fragment from protein G can be immobilized and purified by HSA-Sepharose.

### EXAMPLE IV

### A dual expression system for the generation, analysis and purification of antibodies to a repeated sequence of the Plasmodium falciparum antigen Pf155/RESA¹

A synthetic gene fragment encoding a tetramer of the C-terminal octapeptide repeat EENVEHDA of the Plasmodium falciparum merozoite antigen Pf155/RESA was derived from a construction in the expression vector pATH11 (Aslund L., Sjölander A., Wahlgren M., Wahlin B., Ruangjirachuporn W., Berzins K., Wigzell H., Perlmann P. and Pettersson U. 1987. Synthetic gene construct expressing a repeated and highly immunogenic epitope of the Plasmodium falciparum antigen Pf155. Proc.Natl.Acad.Sci.USA 84:1399). The gene fragment was cut out with the restriction enzymes Xma I and HindIII and ligated into plasmid pEZZT308 (described in Example II) previously digested with the same enzymes. The expression vector pEZZT308 encodes a divalent synthetic IgG-binding domain derived from SpA, preceded by the transcription, translation and secretion signals of the SpA operon. The resulting vector, pEZZM1, encodes a fusion protein consisting of a divalent IgG-binding domain and the octapeptide repeat. This fusion protein was designated ZZ-M1.

The gene fragment, encoding the EENVEHDA repeat was cut out from plasmid pEZZM1 using restriction enzymes Eco RI and HindIII. The same enzymes were used to cut plasmid pB1B2HIVmp18 (described in Example III), into which the fragment was ligated. The resulting vector, pB1B2M1 encodes a fusion protein, designated BB-M1, consisting of the albumin binding region from streptococcal protein G and the octapeptide repeat.

E.coli cells harboring the different constructs were grown overnight at 37°C in 500 ml Tryptic Soy Broth (30 g/l) supplemented with ampicillin (70 mg/l). Cells were pelleted at 6000g and periplasmic proteins were released by osmotic shock. Preparations from the two cultures were applied directly on columns of 5 ml of IgG-Sepharose (Pharmacia, Sweden) or HSA-Sepharose (see Example I).

Analysis of the total periplasmic content by SDS-PAGE indicated that the recombinant product was the major protein produced in both expression systems (Fig. 6, lanes 1 and 3).

New Zealand white rabbits were immunized intramuscularly with 100»g of the fusion protein ZZ-M1 in CFA or without adjuvant. For booster injections, given 4 and 30 weeks later, IFA was used instead of CFA. The rabbits were bled 3 weeks after the first injection and one week after each booster. Antisera from bleeding in week 31 were used. IgG was isolated on a protein A-Sepharose column (Pharmacia).

The capacity of BB-M1 to bind specific antibodies was measured in ELISA. Polystyrene microtiter-plates (Dynatech, Alexandria, Va, USA) were coated with HSA (10 »g/ml) and BB-M1 was allowed to bind to the HSA for 3 h at 37°C. Control plates were coated with the protein G-derived part of BB-M1. After blocking with 0.5% BSA in PBS for 3 h at 37°C, the coated plates were incubated for 1 h at 37°C with various dilutions of the rabbit antiserum obtained from the immunized rabbit. After incubation for 1 h at 37°C with sheep anti-rabbit IgG conjugated with alkaline phosphatase and for 30 minutes at room temperature with p-nitrophenyl phosphate, the resulting color was registered at 405 nm.

The concentration of BB-M1 could be decreased to 200 ng/ml without reducing the antibody-binding capacity of the antigen, and 8 ng/ml of BB-M1 was sufficient to detect reactive antibodies (see Fig. 7).

The reactivity of the rabbit antisera with the EENVEHDA repeat was analyzed in ELISA, using the fusion protein BB-M1 at a concentration of 1000 ng/ml as coating antigen. As shown in Fig. 8, the rabbits immunized with ZZ-M1 in CFA or without adjuvant produced antibodies that reacted with the EENVEHDA repeat in this assay.

Binding of the fusion protein BB-M1 to immobilized HSA might provide an efficient system for affinity purification of peptide specific antibodies. The immobilized HSA may function as a spacer, and the binding of the fusion protein, mediated by the protein G-derived part of BB-M1, should not affect the antibody binding capacity of the EENVEHDA repeat. To test this hypothesis purified BB-M1 was bound to HSA coupled to CNBr-activated Sepharose (Pharmacia) (3.8 mg BB-M1 per 15 mg albumin per ml of packed beads) and crosslinked to HSA by glutaraldehyde (final concentration 0.5%). The reaction was stopped by addition of 1% glycine. The gel was extensively washed in consecutive steps with PBS, 0.2 M acetic acid, pH 3.3, and 0.2 M glycine buffer, pH 2.8. A high yield of covalently bound BB-M1 was obtained using this procedure. Less than 15% of the applied BB-M1 could be eluted from the matrix by extensive washing at low pH. For affinity chromatography, 2 mg of the IgG-fraction isolated from rabbit serum was incubated with 1 ml of packed beads applied to a column. After extensive washing with PBS, bound antibodies were eluted with 0.2 M glycine buffer followed by elution with 3 M KSCN. The buffer of the eluates was changed to PBS containing 0.2% BSA on a PD10 column (Pharmacia) according to the instructions of the manufacturer.

The total IgG, the flow-through fraction and the fraction eluted with 0.2 M glycine buffer from rabbit antisera were tested in ELISA for reactivity with BB-M1. Figure 9 shows the results of an experiment, in which serum from a rabbit immunized with ZZ-M1 in CFA was used for affinity purification. No antibody reactivity with BB-M1 was detected in the flow-through fraction. Less than 0.1% of the applied IgG was eluted with 0.2 m glycine buffer (data not shown). As shown in Fig. 6 a high yield of the BB-M1 reactivity was recovered in the glycine-fraction.

These results demonstrate that the albumin-binding domains of SPG can be used to specifically immobilize a fusion protein including a recombinant peptide to a solid support containing HSA. The immobilized peptide can further be used for analysis and purification of antibodies towards the peptide. A diagramatic scheme of the concept described in this example is outlined in Fig. 10.

## Claims

1. A method of producing and isolating a fusion protein or polypeptide capable of selective binding to serum albumin, characterized by the steps:
a) constructing a recombinant vector comprising a first DNA sequence coding for a serum albumin binding polypeptide fragment and operatively linked to a second DNA sequence coding for a desired protein or polypeptide;
b) transforming a compatible host with said recombinant vector such that the combined DNA sequence coding for said fusion protein or polypeptide can be expressed by the host, and culturing the transformed host in a suitable growth medium to produce said fusion protein or polypeptide; and
c) isolating said fusion protein or polypeptide by making use of its serum albumin binding capacity.

2. A method according to claim 1, wherein the isolation of said protein or polypeptide is performed by adsorption to a carrier material containing serum albumin and optionally desorption of the protein or polypeptide from said carrier material.

3. A method according to claim 1 or 2, wherein said first DNA sequence is constituted by a DNA sequence originating from the gene coding for protein G.

4. A method according to claim 3, wherein said first DNA sequence is comprised by at least one of segments A1, B1, A2, B2, A3 as shown in Figure 1.

5. A method according to claim 4, wherein said first DNA sequence comprises segments B2 and A3 as shown in Figure 1.

6. A method according to any of claims 2 to 5, wherein the desorption of the fusion protein or polypeptide from the carrier material is effected by lowering the pH below about 3.

## Patentansprüche

1. Verfahren zur Herstellung und Isolierung eines Fusionsproteins oder Polypeptids, das in der Lage ist, selektiv an Serumalbumin zu binden, gekennzeichnet durch die Stufen, in denen man
a) einen rekombinanten Vektor aufbaut, der eine erste für ein Serumalbuminbindungs-Polypeptidfragment kodierende erste DNA-Sequenz umfaßt, die funktionsmäßig mit einer für ein erwünschtes Protein oder Polypeptid kodierenden zweiten DNA-Sequenz verbunden ist,
b) einen verträglichen Wirt mit dem rekombinanten Vektor derart transformiert, daß die für das Fusionsprotein oder Polypeptid kodierende kombinierte DNA-Sequenz durch den Wirt exprimiert werden kann, und den transformierten Wirt in einem geeigneten Wachstumsmedium züchtet, um das Fusionsprotein oder Popypeptid zu erzeugen, und
c) das Fusionsprotein oder Polypeptid isoliert, indem man sich seine Serumalbuminbindungskapazität zunutze macht.

2. Verfahren nach Anspruch 1, bei dem die Isolierung des Proteins oder Polypeptids durch Adsorption an einem Serumalbumin enthaltenden Trägermaterial und gegebenenfalls Desorption des Proteins oder Polypeptids von dem Trägermaterial erfolgt.

3. Verfahren nach Anspruch 1 oder 2, bei dem die erste DNA-Sequenz von einer DNA-Sequenz gebildet wird, die aus dem für Protein G kodierenden Gen stammt.

4. Verfahren nach Anspruch 3, bei dem die erste DNA-Sequenz wenigstens eines der Segmente A1, B1, A2, B2, A3, wie in Fig. 1 gezeigt, umfaßt.

5. Verfahren nach Anspruch 4, bei dem die erste DNA-Sequenz Segmente B2 und A3, wie in Fig. 1 gezeigt, umfaßt.

6. Verfahren nach einem der Ansprüche 2 bis 5, bei dem die Desorption des Fusionsproteins oder Polypeptids von dem Trägermaterial durch Senkung des pH-Wertes unter etwa 3 bewirkt wird.

## Revendications

1. Procédé de production et d'isolement d'une protéine ou d'un polypeptide de fusion capable de se lier de manière sélective à une sérum-albumine, caractérisé par les étapes qui consistent à:
a) construire un vecteur recombinant comprenant une première séquence d'ADN codant pour un fragment de polypeptide fixant la sérum-albumine et liée de manière fonctionnelle à une deuxième séquence d'ADN codant pour une protéine ou un polypeptide souhaité;
b) transformer un hôte compatible avec ledit vecteur recombinant afin que la séquence d'ADN combinée codant pour ladite protéine ou ledit polypeptide de fusion puisse être exprimée par l'hôte, et cultiver l'hôte transformé dans un milieu de culture convenable pour produire ladite protéine ou ledit polypeptide de fusion; et
c) isoler ladite protéine ou ledit polypeptide de fusion en utilisant sa capacité de fixation de la sérum-albumine.

2. Procédé selon la revendication 1, dans lequel l'isolement de ladite protéine ou dudit polypeptide est effectué par adsorption sur une matière véhicule contenant de la sérum-albumine et éventuellement désorption de la protéine ou du polypeptide à partir de ladite matière véhicule.

3. Procédé selon la revendication 1 ou 2, dans lequel ladite première séquence d'ADN est constituée par une séquence d'ADN provenant du gène codant pour la protéine G.

4. Procédé selon la revendication 3, dans lequel ladite première séquence d'ADN est constituée par au moins un des segments A1, B1, A2, B2, A3 comme le montre la figure 1.

5. Procédé selon la revendication 4, dans lequel ladite première séquence d'ADN comprend les segments B2 et A3 comme le montre la figure 1.

6. Procédé selon l'une quelconque des revendications 2 à 5, dans lequel la désorption de la protéine ou du polypeptide de fusion à partir de la matière véhicule est réalisée en abaissant le pH en dessous d'environ 3.
